# EUROPEAN PATENT SPECIFICATION

(11) **EP 1 913 049 B1**
(45) Date of publication and mention of the grant of the patent: **22.07.2009**
(21) Application number: 06800369.8
(22) Date of filing: 26.07.2006
(51) Int. Cl.: C08G 18/36, C08G 18/38, C07C 67/26, C08J 9/04

(54) **POLYURETHANES MADE FROM HYDROXYL-CONTAINING FATTY ACID AMIDES**
AUS FETTSÄUREAMIDEN MIT HYDROXYL HERGESTELLTE POLYURETHANE
POLYURETHANNES CONÇUS A PARTIR D'AMIDES D'ACIDES GRAS RENFERMANT DE L'HYDROXYLE

(30) Priority: 03.08.2005 US 705086 P
(43) Date of publication of application: 23.04.2008
(73) Proprietor: DOW GLOBAL TECHNOLOGIES INC., Midland MI 48674 (US)
(72) Inventor: MARTIN, Charles, A., Pearland, TX 77584 (US); SANDERS, Aaron, W., Missouri City, TX 77459 (US); LYSENKO, Zenon, Midland, MI 48640 (US); SCHROCK, Alan, K., Lake Jackson, TX 77566 (US); BABB, David, Alan, Lake Jackson, TX 77566 (US); EARLS, Jim, D., Lake Jackson, TX 77566 (US); OLSON, Kurt, D., Cross Lanes, WV 25313 (US); BRIGGS, John, R., Midland, MI 48640 (US)
(74) Representative: Raynor, John
(86) International application number: PCT/US2006/029085
(87) International publication number: WO 2007/019063

(56) References cited:
- EP-A2- 0 554 590
- US-A- 3 637 539
- US-A- 3 699 061
- KHOE T H ET AL: "POLYURETHANE FOAMS FROM HYDROXYMETHYLATED FATTY DIETHANOLAMIDES" JOURNAL OF THE AMERICAN OIL CHEMISTS' SOCIETY, AOCS PRESS, CHAMPAIGN, IL, US, vol. 50, 1973, pages 331-333, XP009075832 ISSN: 0003-021X cited in the application

## Description

This invention relates to polyurethane polymers and methods for making such polymers.

Polyurethanes are produced by the reaction of polyisocyanates and polyols. One type of polyurethane, rigid polyurethane foam, is widely used in thermal insulation and structural applications. The starting materials used to make these rigid polyurethane foams tend to be low equivalent weight, high functionality polyols and high functionality polyisocyanates, as these materials provide a densely crosslinked polymeric structure. The polyols are most typically polyethers and polyesters that are derived from petroleum feedstocks. Rigid polyurethane foams have been made with castor oil or castor oil byproducts.

Polyols for rigid foam applications must meet several demands. They provide the needed crosslinking to the polymer structure and to form a foam having the necessary mechanical attributes. The polyols must react with the other components in the formulation to form a foam having a fine and uniform cell structure. This is especially the case when the foam is used in thermal insulating applications. To accomplish this, the polyols must be reasonably compatible with the other components in the formulation, in particular water and the polyisocyanate. It is especially desirable that the polyol can be used with readily available surfactants and catalyst packages. The polyols should be reactive enough that the foam rises and cures quickly without the need for very high levels of catalysts, while still providing for good processing and yielding a high quality foam.

Because of unpredictable crude oil pricing and a growing desire to find alternative feedstocks for making commodity chemicals, there is an interest in replacing conventional polyols with newer materials that are made using renewable feedstocks such as vegetable oils or animal fats.

One approach to creating vegetable oil-based polyols is described in EP 0 106 491A2. Certain fatty acid mixtures are hydroxymethylated, and esters are formed by reacting the hydroxymethylated material with a polyhydroxyl initiator. More recently, higher functionality versions of these materials have been developed, as described in WO 04/096882A and WO 04/096883A. These polyols are described as being useful in flexible foam and other elastomeric polyurethane applications.

Amides of hydroxymethylated fatty acids with alkanolamines have been described for use in making rigid polyurethane foam. *See* Khoe et al., "Polyurethane Foams form Hydroxymethylated Fatty Diethanolamides", J. Amer. Oil Chemists' Society 50:331-333 (1973). The foam described therein was made using Freon 11 as a blowing agent. Khoe et al. report that in such a formulation, the amide compound produced foam with inadequate dimensional stability when used as the sole polyol.

Other vegetable oil-based polyols are described, for example in GB1248919. These polyols are prepared in the reaction of a vegetable oil with an alkanolamine (such as triethanolamine) to form a mixture of monoglycerides, diglycerides, and reaction products of the alkanolamine and fatty acid groups from the vegetable oil. These materials have free hydroxyl groups on the glycerine and alkanolamine portions of the molecules. The free hydroxyl groups are ethoxylated to increase reactivity and to provide a somewhat more hydrophilic character. This makes the product more compatible with a foam formulation containing water as a blowing agent. These products tend to have hydroxyl numbers in the range of from 185 to 200, which corresponds to a hydroxyl equivalent weight in the range of about 280 to 305, and a functionality of about 2.3. The equivalent weights tend to be higher than preferred and the functionalities are lower than needed for producing good quality rigid polyurethane foam.

It would be desirable to provide a polyol that is based on annually renewable feedstocks such as vegetable oils, which can be used to make good quality rigid polyurethane foams. It would be desirable to provide a polyurethane foam that is made using a significant proportion of raw materials derived from an annual renewable feedstock.

In one aspect, this invention is a process for preparing a polyurethane, comprising
(a) forming a reaction mixture by mixing a polyol or mixture thereof with a polyisocyanate compound, wherein the polyol or polyol mixture includes one or more compounds having (1) an amide group having at least one hydroxyl-containing organic group bonded to the nitrogen atom of the amide group and (2) a branched or straight chain C₇₋₂₃ hydrocarbon group bonded directly to the carbonyl carbon of the amide group wherein the C₇₋₂₃ hydrocarbon group is substituted with at least one (i) (N-hydroxyalkyl) amino alkyl group or (ii) hydroxyl-containing ester group; and
(b) subjecting the reaction mixture to conditions such that it cures to form a polyurethane.

In another aspect, this invention is a polyurethane made by the foregoing process. In a preferred such process, the polyurethane is a rigid polyurethane foam, the reaction mixture includes a blowing agent and a surfactant, and polyol or mixture thereof has an average hydroxyl equivalent weight of from 100 to 350 and an average hydroxyl functionality of at least 2.5.

In another aspect, the invention is a rigid polyurethane foam made by the foregoing process.

In yet another aspect, this invention is a polyol which is useful in making a polyurethane, and in particular a rigid polyurethane, in accordance with the invention. The polyol is a compound that includes (1) an amide group having at least one hydroxyalkyl group bonded to the nitrogen atom of the amide group, and (2) a branched or straight chain C₇₋₂₃ hydrocarbon group bonded directly to the carbonyl carbon of the amide group. At least one hydroxyl-containing ester group is bonded to the C₇₋₂₃ hydrocarbon group. The polyol of the invention can be prepared in alternative ways as described more below.

In making a polyurethane in accordance with the invention, a polyol or polyol mixture is reacted with an organic polyisocyanate. In embodiments of particular interest, the polyurethane is a rigid foam, and the polyol or polyol mixture has an average hydroxyl equivalent weight of from 100 to 350, preferably from 100 to 250 and especially from 110 to 150. For rigid foam applications, the polyol or polyol mixture contains one or more polyols that in the aggregate have an average hydroxyl functionality of at least 2.5, especially from 2.8 to 6 and most preferably from 3.0 to 4.5.

In one aspect of the invention, at least one polyol used in making the polyurethane is an amide compound having at least one amide (>N-C(O)-) group. This amide compound has at least one hydroxyl-containing organic group bonded to the nitrogen atom of the amide group. The compound further has a branched or straight chain C₇₋₂₃ hydrocarbon group bonded directly to the carbonyl carbon of the amide group. The C₇₋₂₃ hydrocarbon group is substituted with at least one N-hydroxyalkyl aminoalkyl group or hydroxyl-containing ester group. These amide compounds are conveniently prepared in several steps using vegetable oils or animal fats, or unsaturated fatty acids obtained from vegetable oils or animal fats, as a starting material.

The amide compound will typically be a mixture of materials having on average from one to eight or more hydroxyl groups per molecule. A preferred mixture of amide compounds contains on average at least two, especially at least 2.5 hydroxyl groups/molecule. A mixture of amide compounds having on average from 3 to 6 hydroxyl groups/molecule is especially preferred.

### Amide compounds containing at least one (N-hydroxyalkyl) aminoalkyl group substituted onto a fatty acid chain

This type of amide compound is conveniently described as an amide of a fatty acid (or ester) and a hydroxyl-containing primary or secondary amine, in which the fatty acid group has been modified to introduce one or more (N-hydroxyalkyl) aminoalkyl groups. These materials can be prepared from vegetable or oils or animal fats in a sequence of reactions, as follows.

An unsaturated fatty acid ester, or mixture thereof with other saturated or unsaturated fatty acid esters, is used as a starting material. These can be produced from the vegetable oil or animal fat in the manner described before. As before, at least 50 mole% and more preferably at least 80 mole% of the fatty acids contains at least one site of carbon-carbon unsaturation. As before, the fatty acids may contain two or more of such sites, in which case those sites preferably are not conjugated.

An amide is prepared from the fatty acid ester by reacting it with a primary or secondary amine compound that contains at least one hydroxyl-containing organic group. Suitable conditions for conducting this reaction are described before. Suitable amine compounds are those described earlier with respect to the hydroxymethyl group-containing amide compounds.

N-hydroxyalkylamino groups can be introduced onto the fatty acid chain of the resulting amide at sites of carbon-carbon unsaturation, via a reaction with a mono- or dialkanolamine in the presence of carbon monoxide and hydrogen gas. Synthetic methods for performing such a reductive amination reaction are described, for example, in Science 2002, 297, 1676-1678 and in United States Provisional Application No. 60/565781, filed April 27, 2005. Preferred alkanolamines for use in this reductive amination step include diethanolamine, monoethanolamine, di(isopropanol)amine, monoisopropanolamine, and the like. Dialkanolamines are generally more preferred as they result in a higher functionality product. Mixtures of dialkanolamines or of monoalkanolamines with dialkanolamines may also be used.

In a preferred method, as described in United States Provisional Application No. 60/565781, the reductive amination is conducted in the presence of a neutral rhodium-monodentate phosphite ligand complex. The neutral rhodium-monodentate phosphite ligand complex is conveniently prepared by contacting a neutral rhodium procatalyst with a stoichiometric excess of a monodentate phosphite ligand in the presence of a solvent such as dioxane, THF, cyclohexane, toluene, acetone, or o-xylene. The monodentate phosphite ligand can be characterized by the general formula P(OR)₃, where each R is independently a carbon-containing substituent. Preferably, each R independently includes an alkyl, aryl, arylalkyl, arylalkoxy, or carbonylaryl group. Examples of representative monodentate phosphites include triphenylphosphite, tris(2,4-di-t-butylphenyl)phosphite, tri-o-tolylphosphite, tri-p-tolylphosphite, trimethylphosphite, triethylphosphite, tri-n-propylphosphite, tri-n-butylphosphite, tri-t-butylphosphite, tri-1-naphthylphosphite, tri-2-naphthylphosphite, 2,2-biphenolphenylphosphite, 2,2',4,4'-tetra-t-butyl-2,2'-biphenol 2,4-di-t-butylphenylphosphite, and tribenzylphosphite.

The neutral rhodium procatalyst is a rhodium (I) catalyst precursor characterized by having its positive charge balanced by the negative charge of supporting bound ligands. For example, rhodium dicarbonyl acetonylacetate is a neutral rhodium procatalyst and, therefore, suitable. Other suitable examples of neutral rhodium procatalysts useful in preparing the complex include [Rh₄(CO)₁₂], [Rh₂(OAc)₄], [Rh(C₂H₄)₂(acac)], [Rh(cyclooctadiene)(acac)], [(Rh(norbornene)₂(acac)], [(Rh(norbornadiene)(acac)], and [Rh(acac)₃] procatalysts.

The amide, alkanolamine and catalyst complex are advantageously saturated with a stoichiometric excess of a mixture of CO and H₂ and subject to an elevated temperature and pressure. The mole-to-mole ratio of CO:H₂ is suitably from about 1:1 to about 4:1. The reaction is carried out at a pressure of not less than 200 psi (1380 kPa) to as high as 3000 psi (20700 kPa). A preferred reaction temperature is from 20 to 120°C.

This reaction product contains a mono- or di(hydroxyalkyl)-substituted aminomethyl group at a site of carbon-carbon unsaturation in the fatty acid chain. If the fatty acid material contains more than one site of carbon-carbon unsaturation, multiple such aminomethyl groups are usually introduced. In addition, the amide will contain one or more alkanol groups attached to the amide nitrogen atom. Amides of this type advantageously have an average hydroxyl functionality of at least 2.5, especially at least 2.8 and more preferably at least 3.0.

### Amide compounds having pendant hydroxyl-substituted ester groups.

This type of amide compound is conveniently prepared by (1) forming an amide of an unsaturated fatty acid and a hydroxyl-containing primary or secondary amine, (2) epoxidizing the unsaturated fatty acid group and then (3) reacting the resulting epoxy group with a hydroxy acid or a hydroxy acid precursor. A hydroxyl-substituted group is formed, which is bound to the fatty acid chain through an ester linkage.

The hydroxyl-containing primary or secondary amine is as described before. The unsaturated fatty acid is preferably used in the form of a lower alkyl ester, which is conveniently prepared from vegetable oils or animal fats as described before. As before, mixtures of unsaturated fatty acids or of unsaturated fatty acids with saturated fatty acids can be used if desired. Conditions for the amide-forming reaction are suitably as described before.

Sites of carbon-carbon unsaturation in the fatty acid chain are then epoxidized. Conditions for epoxidizing carbon-carbon double bonds are well known, and commonly include the use of organic peresters, organic peracids or organic peroxides as reagents. It is preferred to epoxidize at least 50%, especially at least 80% and most preferably at least 95% of the carbon-carbon double bonds in the fatty acid groups.

The epoxide group is then reacted with a hydroxy acid or hydroxy acid precursor to introduce a hydroxy-substituted ester group onto the fatty acid chain. The ester group will be introduced at the locus of the epoxy group(s). The size of the pendant ester group will depend on the selection of the hydroxy acid. The pendant ester group preferably contains from 3 to 10, especially from 3 to 6 carbon atoms. Suitable hydroxy acids and esters include lactic acid, glycolic acid, 2,2-dimethylolpropionic acid. A hydroxy acid precursor that produces the hydroxy acid under the conditions of the reaction can be used instead or in conjunction with the hydroxy acid. Examples of such precursors are cyclic dianhydride dimers of the hydroxy acid, such as lactide and glycolide. These materials can be reacted with epoxy groups on the fatty acid amide using a wide range of catalysts and conditions as are commonly used in esterification reactions. Those conditions generally include an elevated temperature, suitably of from 80 to 200°C. Suitable catalysts include a wide range of acid and strong Lewis acid compounds, as these favor the ring-opening reaction of the hydroxyacid with the epoxide. When a hydroxy-substituted acid is used as a starting material, it may be desirable to conduct the reaction under a reduced pressure, to remove water as it forms during the condensation reaction.

This reaction product contains one or hydroxy-substituted ester groups pendant from the fatty acid portion of the amide. In addition, the product amide compound will contain one or more alkanol groups attached to the amide nitrogen atom. Amide compounds of this type advantageously have an average hydroxyl functionality of at least 2.0, especially at least 2.5 and more preferably at least 2.8.

### Polyurethanes, especially rigid polyurethane foams, made using the foregoing amides

The foregoing amide compounds are capable of reacting with an organic polyisocyanate to form a variety of polyurethanes. The types of polyurethane that can be prepared include rigid foam, flexible foam, molded elastomers, adhesives, sealants, The amide compound may be used as the sole polyol in the polyurethane-producing reaction, or may be combined with other polyols to achieve a desired set of properties in the polyurethane. The amide compound may be used as already described. Alternatively, its equivalent weight may be increased by alkoxylating the hydroxyl groups with an alkylene oxide such as ethylene oxide, propylene oxide, butylene oxide or tetrahydrofuran.

Rigid polyurethane foam is an application of particular interest, as the amide compound of the invention can be prepared with a high hydroxyl functionality. In addition, the presence of nitrogen atoms in the amide causes it to be somewhat autocatalytic, which is often a desirable trait for rigid foam polyols.

Rigid polyurethane foam is prepared using the amide compounds described above by forming a polyol component containing the amide compound, and contacting the polyol component with at least one polyisocyanate compound in the presence of a blowing agent and a surfactant. The resulting reaction mixture is subjected to conditions at which the polyol(s) react with the polyisocyanate and a gas is generated to expand the reacting mixture and form a foam.

The polyol component includes at least one amide compound as described. The amide compound may constitute the sole polyol in the polyol component. Alternatively, other polyols may be used in combination with the amide compound in order to achieve a desired reactivity or level of crosslinking, or otherwise to achieve a particular set of properties in the foam. When the amide compound is used with other polyols, the amide compound can constitute as little as one percent by weight of the polyols. Preferably, the amide compound constitutes from 10 to 100% of the polyols (by weight), such as from 30 to 100%, from 50 to 100% or from 75 to 100% thereof.

Suitable polyols (other than the amide compound) are compounds having at least two isocyanate-reactive hydroxyl groups per molecule. The functionality of the individual polyols preferably ranges from about 2 to about 12, more preferably from about 2 to about 8. The hydroxyl equivalent weight of the individual polyols may range from about 31 to about 2000 or more. However, for preparing rigid polyurethanes, the equivalent weight of the polyols is selected in conjunction with that of the amide compound so the polyol component as a whole has an equivalent weight is from about 100 to 150, preferably from about 100 to 250 and especially from about 110 to about 150. Preferably, the hydroxyl equivalent weight of the individual polyols is from about 31 to about 500, more preferably from about 31 to about 250, even more preferably from about 31 to about 200.

Suitable additional polyols include compounds such as alkylene glycols (e.g., ethylene glycol, propylene glycol, 1,4-butane diol, 1,6-hexanediol and the like), glycol ethers (such as diethylene glycol, triethylene glycol, dipropylene glycol, tripropylene glycol and the like), glycerine, trimethylolpropane, tertiary amine-containing polyols such as triethanolamine, triisopropanolamine, and ethylene oxide and/or propylene oxide adducts of ethylene diamine, toluene diamine and the like, polyether polyols, polyester polyols. Among the suitable polyether polyols are polymers of alkylene oxides such as ethylene oxide, propylene oxide and 1,2-butylene oxide or mixtures of such alkylene oxides. Preferred polyethers are polypropylene oxides or polymers of a mixture of propylene oxide and a small amount (up to about 12 weight percent) ethylene oxide. These preferred polyethers can be capped with up to about 30% by weight ethylene oxide.

Polyester polyols are also suitable additional polyols. These polyester polyols include reaction products of polyols, preferably diols, with polycarboxylic acids or their anhydrides, preferably dicarboxylic acids or dicarboxylic acid anhydrides. The polycarboxylic acids or anhydrides may be aliphatic, cycloaliphatic, aromatic and/or heterocyclic and may be substituted, such as with halogen atoms. The polycarboxylic acids may be unsaturated. Examples of these polycarboxylic acids include succinic acid, adipic acid, terephthalic acid, isophthalic acid, trimellitic anhydride, phthalic anhydride, maleic acid, maleic acid anhydride and fumaric acid. The polyols used in making the polyester polyols preferably have an equivalent weight of about 150 or less, especially 75 or less, and include ethylene glycol, 1,2- and 1,3-propylene glycol, 1,4- and 2,3-butane diol, 1,6-hexane diol, 1,8-octane diol, neopentyl glycol, cyclohexane dimethanol, 2-methyl-1,3-propane diol, glycerine, trimethylol propane, 1,2,6-hexane triol, 1,2,4-butane triol, trimethylolethane, pentaerythritol, quinitol, mannitol, sorbitol, methyl glycoside, diethylene glycol, triethylene glycol, tetraethylene glycol, dipropylene glycol, dibutylene glycol. Polycaprolactone polyols such as those sold by The Dow Chemical Company under the trade name "Tone" are also useful.

Preferred additional polyols are alkylene glycols, glycol ethers of up to about 75 equivalent weight, glycerine, trimethylolpropane, triethanolamine, triisopropanolamine, and poly(propylene oxide) polyols of up to about 200 equivalent weight.

The additional polyol (when used) may include a tertiary amine-containing polyol and/or an amine-functional compound. Such tertiary amine-containing polyols include, for example, triisopropanol amine, triethanolamine and ethylene and/or propylene oxide adducts of ethylene diamine, toluene diamine or aminoethylpiperazine having a molecular weight of up to about 800, preferably up to about 400.

The amine-functional compound is a compound having at least two isocyanate-reactive groups, of which at least one is a primary or secondary amine group. Among these are monoethanolamine, diethanolamine, monoisopropanol amine, diisopropanol amine and aliphatic polyamines such as aminoethylpiperazine. Also included among these compounds are the so-called aminated polyethers in which all or a portion of the hydroxyl groups of a polyether polyol are converted to primary or secondary amine groups. Suitable such aminated polyethers are sold by Huntsman Chemicals under the trade name JEFFAMINE®. Typical conversions of hydroxyl to amine groups for these commercial materials range from 70 to 95%, and thus these commercial products contain some residual hydroxyl groups in addition to the amine groups. Preferred among the aminated polyethers are those having a weight per isocyanate-reactive group from 100 to 1700, especially from 100 to 250, and having from 2 to 4 isocyanate-reactive groups per molecule.

In order to impart toughness to the foam, a minor amount of a high (i.e. 800 or higher, preferably from 1500 to 3000) equivalent weight polyol may be added to the polyol component. This high equivalent weight polyol is preferably a polyether polyol having two to three hydroxyl groups per molecule. It more preferably is a poly(propylene oxide) that may be end-capped with up to 30% (by weight of the polyol) of poly(ethylene oxide). The high equivalent weight polyol may contain dispersed polymer particles. These materials are commercially known and are commonly referred to as "polymer polyols" (or, sometimes "copolymer polyols"). The dispersed polymer particles may be, for example, polymers of a vinyl monomer (such as styrene, acrylonitrile or styrene-acrylonitrile particles), polyurea particles or polyurethane particles. Polymer or copolymer polyols containing from about 2 to about 50% or more by weight dispersed polymer particles are suitable. When used, this polymer or copolymer polyol may constitute up to about 45%, preferably from about 5 to about 40%, of the weight of all isocyanate-reactive materials in the polyol component.

Suitable polyisocyanates include aromatic, cycloaliphatic and aliphatic isocyanates. Exemplary polyisocyanates include m-phenylene diisocyanate, toluene-2-4-diisocyanate, toluene-2-6-diisocyanate, isophorone diisocyanate, 1,3- and/or 1,4-bis(isocyanatomethyl)cyclohexane (including cis- or trans-isomers of either), hexarriethylene-1,6-diisocyanate, tetramethylene-1,4-diisocyanate, cyclohexane-1,4-diisocyanate, hexahydrotoluene diisocyanate, methylene bis(cyclohexaneisocyanate) (H₁₂MDI), naphthylene-1,5-diisocyanate, methoxyphenyl-2,4-diisocyanate, diphenylmethane-4,4'-diisocyanate, 4,4'-biphenylene diisocyanate, 3,3'-dimethoxy-4,4'-biphenyl diisocyanate, 3,3'-dimethyl-4-4'-biphenyl diisocyanate, 3,3'-dimethyldiphenyl methane-4,4'-diisocyanate, 4,4',4"-triphenyl methane triisocyanate, a polymethylene polyphenylisocyanate (PMDI), toluene-2,4,6-triisocyanate and 4,4'-dimethyldiphenylmethane-2,2',5,5'-tetraisocyanate. Preferably the polyisocyanate is diphenylmethane-4,4'-diisocyanate, diphenylmethane-2,4'-diisocyanate, PMDI, toluene-2-4-diisocyanate, toluene-2-6-diisocyanate or mixtures thereof. Diphenylmethane-4,4'-diisocyanate, diphenylmethane-2,4'-diisocyanate and mixtures thereof are generically referred to as MDI, and all can be used. Toluene-2,4-diisocyanate, toluene-2,6-diisocyanate and mixtures thereof are generically referred to as TDI, and all can be used.

Derivatives of any of the foregoing polyisocyanate groups that contain biuret, urea, carbodiimide, allophonate and/or isocyanurate groups can also be used. These derivatives often have increased isocyanate functionalities and are desirably used when a more highly crosslinked product is desired.

Suitable blowing agents include physical blowing agents such as various low-boiling chlorofluorocarbons, fluorocarbons, hydrocarbons Fluorocarbons and hydrocarbons having low or zero global warming and ozone-depletion potentials are preferred among the physical blowing agents. Chemical blowing agents that decompose or react under the conditions of the polyurethane-forming reaction are also useful.

By far the most preferred chemical blowing agent is water, which reacts with isocyanate groups to liberate carbon dioxide and form urea linkages. Water is preferably used as the sole blowing agent, in which case from 1 to 7, especially from 1.5 to 5 parts by weight water are typically used per 100 parts by weight of isocyanate-reactive materials (exclusive of water). Water may also be used in combination with a physical blowing agent, particularly a fluorocarbon or hydrocarbon blowing agent. In addition, a gas such as carbon dioxide, air, nitrogen or argon may be used as the blowing agent in a frothing process. It has been found that when the blowing agent includes at least one part of water per 100 parts by weight of isocyanate-reactive materials (exclusive of water), dimensionally stable foams can be produced even when the amide compound constitutes a high proportion, such as from 80 to 100% or from 90 to 100% by weight, of the isocyanate-reactive materials (exclusive of water).

A wide variety of silicone surfactants as are commonly used in making polyurethane foams can be used in making the foams in accordance with this invention. Examples of such silicone surfactants are commercially available under the tradenames Tegostab^{™} (Th. Goldschmidt and Co.), Niax^{™} (GE OSi Silicones) and Dabco^{™} (Air Products and Chemicals).

The reaction mixture may contain a wide variety of other additives as are conventionally used in making polyurethanes of various types. These include, for example, catalysts, blowing agents, surfactants, cell openers, fillers such as calcium carbonate; pigments and/or colorants such as titanium dioxide, iron oxide, chromium oxide, azo/diazo dyes, phthalocyanines, dioxazines and carbon black; reinforcing agents such as fiber glass, carbon fibers, flaked glass, mica, talc and the like, biocides, preservatives, antioxidants, flame retardants. Catalysts are particularly preferred additives, as are blowing agents and surfactants in cases where a cellular polyurethane is desired.

A catalyst is often used to promote the polyurethane-forming reaction. The selection of a particular catalyst package will vary somewhat with the particular application, the particular polyol(s) being used, and the other ingredients in the formulation. The catalyst may catalyze the "gelling" reaction between the polyol(s) and the polyisocyanate and/or, in many polyurethane foam formulation(s), the water/polyisocyanate (blowing) reaction which generates urea linkages and free carbon dioxide to expand the foam.

A wide variety of materials are known to catalyze polyurethane forming reactions, including tertiary amines, tertiary phosphines, various metal chelates, acid metal salts, strong bases, various metal alcoholates and phenolates and metal salts of organic acids. Catalysts of most importance are tertiary amine catalysts and organotin catalysts. Examples of tertiary amine catalysts include trimethylamine, triethylamine, N-methylmorpholine, N-ethylmorpholine, N,N-dimethylbenzylamine, N,N-dimethylethanolamine, N,N,N',N'-tetramethyl-1,4-butanediamine, N,N-dimethylpiperazine, 1,4-diazobicyclo-2,2,2-octane, bis(dimethylaminoethyl)ether, triethylenediamine and dimethylalkylamines where the alkyl group contains from 4 to 18 carbon atoms. Mixtures of these tertiary amine catalysts are often used. Examples of suitably commercially available surfactants include Niax^{™} A1 (bis(dimethylaminoethyl)ether in propylene glycol available from GE OSi Silicones), Niax^{™} B9 (N,N-dimethylpiperazine and N-N-dimethylhexadecylamine in a polyalkylene oxide polyol, available from GE OSi Silicones), Dabco^{™} 8264 (a mixture of bis(dimethylaminoethyl)ether, triethylenediamine and dimethylhydroxyethyl amine in dipropylene glycol, available from Air Products and Chemicals), and Dabco^{™} 33LV (triethylene diamine in dipropylene glycol, available from Air Products and Chemicals), Niax^{™} A 400 (a proprietary tertiary amine/carboxylic salt and bis (2-dimethylaminoethy)ether in water and a proprietary hydroxyl compound, available from GE OSi Silicones); Niax^{™} A-300 (a proprietary tertiary amine/carboxylic salt and triethylenediamine in water, available from GE OSi Specialties Co.); Polycat^{™} 58 (a proprietary amine catalyst available from Air Products and Chemicals), Polycat^{™} 5 (pentamethyl diethylene triamine, available from Air Products and Chemicals) and Polycat^{™} 8 (N,N-dimethyl cyclohexylamine, available from Air Products and Chemicals).

Examples of organotin catalysts are stannic chloride, stannous chloride, stannous octoate, stannous oleate, dimethyltin dilaurate, dibutyltin dilaurate, other organotin compounds of the formula SnRₙ(OR)₄₋ₙ, wherein R is alkyl or aryl and n is 0-2, and the like. Commercially available organotin catalysts of interest include Dabco^{™} T-9 and T-95 catalysts (both stannous octoate compositions available from Air Products and Chemicals).

Catalysts are typically used in small amounts, for example, each catalyst being employed from about 0.0015 to about 5% by weight of the high equivalent weight polyol.

A polyurethane is formed by bringing the components of the reaction mixture together under conditions that they react and form a polyurethane polymer. These reactions usually occur spontaneously upon mixing the polyisocyanate with the polyol component at room temperature or an elevated temperature. Accordingly, no special conditions are needed in most cases to form a polyurethane foam having good properties. The amount of polyisocyanate used is sufficient to provide an isocyanate index, i.e., 100 times the ratio of NCO groups to isocyanate-reactive groups in the reaction mixture (including those provided by water if used as a blowing agent), of from 85 to 300, especially from 95 to 150. The use of an isocyanate index above about 150 favors the formation of isocyanurate groups in the polymer.

The polyurethane foam may be formed as foam-in-place insulation, such as for refrigerators, freezers, coolers, ship hulls. It can also be formed as boardstock foam.

The urethane-forming reactions (as well as the water-isocyanate reaction) often proceed well even at room temperature, and are usually exothermic enough to drive the polyurethane-forming reactions nearly to completion.

The following examples are provided to illustrate the invention, but are not intended to limit the scope thereof. All parts and percentages are by weight unless otherwise indicated.

### Example 1A: Preparation of Amide Compound

9(10)-hydroxymethylstearate (400 parts by weight), diethanolamine (361.5 parts) and an 85% solution of potassium hydroxide in water (0.832 part) are placed in a vessel equipped with stirrer, nitrogen purge and condenser. The flask is heated to 85°C for 22 hours. The reaction mixture is cooled to 27°C and toluene is added to dissolve the amide product. The resulting toluene solution is washed three times with a 2% sodium bicarbonate solution in water. The toluene layer is then dried with anhydrous sodium sulfate and filtered. The dried toluene layer is evaporated under vacuum to remove the toluene. The resulting liquid product is stirred with methanol and filtered, followed by distillation of the methanol from the solution. 467 parts of a product (N,N-bis(hydroxyethyl) hydroxymethylstearamide) containing 12.16% by weight hydroxyl groups (∼140 hydroxyl equivalent weight) is obtained.

### Example 1B: Preparation of Rigid Polyurethane Foam

73.95 parts of N,N-bis(hydroxyethyl) hydroxymethylstearamide (from Example 1A) are blended with 2.96 parts of a catalyst mixture, 1.59 parts of Niax^{™}L-6900 silicone surfactant (GE silicones), 1.91 parts of water and 19.6 parts of HCFC-141b. The catalyst mixture includes pentamethylene diamine (Polycat^{™}5 from Air Products and Chemicals), dimethylcyclohexyl amine (Polycat^{™}8 from Air Products and Chemicals), and a potassium salt in diethylene glycol (PolyCat^{™}46, from Air Products and Chemicals). The resulting mixture is combined at room temperature with 143.23 parts (1.15 index) of a polymeric MDI having a functionality of 2.7 and an isocyanate equivalent weight of 134, and allowed to rise freely and cure.

Gel time, measured from the time the polyol and polyisocyanate components are mixed until such time as the mixture forms strings when a spatula is touched to the mixture and pulled away, is 22 seconds. Tack free time is 23 seconds. Free rise density, measured on a 4" X 4" X 4" (10 cm X 10 cm X 10 cm) cube cut from the center of the cured foam, is 1.1 pounds/cubic foot (∼17.6 kg/m³).

For comparison, a foam is made using the same formulation, except a 360-OH number poly(propylene oxide) polyol (Voranol® 360 from Dow Chemical) is used in replace of the polyol from Example 1A. The gel time is 41 seconds and the tack time is 60 seconds, indicating a somewhat higher reactivity in the formulation of the invention. Density is 1.51 lb/ft³ (∼24 kg/m³).

## Claims

1. A process for preparing a polyurethane, comprising
(a) forming a reaction mixture by mixing a polyol or mixture thereof with a polyisocyanate compound, wherein the polyol or polyol mixture includes one or more compounds having (1) an amide group having at least one hydroxyl-containing organic group bonded to the nitrogen atom of the amide group and (2) a branched or straight chain C₇₋₂₃ hydrocarbon group bonded directly to the carbonyl carbon of the amide group, wherein the C₇₋₂₃ hydrocarbon group is substituted with at least one (i) (N-hydroxyalkyl) amino alkyl group or (ii) hydroxyl-containing ester group; and
(b) subjecting the reaction mixture to conditions such that it cures to form a polyurethane.

2. A polyurethane made by the process of claim 1.

3. The process of claim 1, wherein the reaction mixture further comprises a blowing agent and a surfactant, the polyol or mixture thereof has an average hydroxyl equivalent weight from 100 to 350 and an average hydroxyl functionality of at least 2.5, and the polyurethane is a rigid polyurethane foam.

4. The process of claim 3, wherein the C₇₋₂₃ hydrocarbon group is substituted with at least one (N-hydroxyalkyl) aminoalkyl group.

5. The process of claim 4, wherein at least one hydroxyalkyl group is bonded to the nitrogen atom of the amide group.

6. The process of claim 5, wherein the hydroxyalkyl group bonded to the nitrogen atom of the amide group is a hydroxyethyl group.

7. The process of claim 6, wherein the N-(hydroxyalkyl) aminoalkyl group is an N-(hydroxyalkyl) aminomethyl group.

8. The process of claim 7, wherein the N-hydroxyalkyl aminomethyl group is a N-(hydroxyethyl) aminomethyl group or an N,N-bis(hydroxyethyl) aminomethyl group.

9. The process of claim 8, wherein two hydroxyethyl groups are bonded to the nitrogen atom of the amide group.

10. The process of claim 9, wherein the amide compound is formed by first reacting an unsaturated fatty acid or ester thereof with an alkanolamine mixture containing diethanolamine and monoethanolamine to form an amide compound, and then reacting the amide compound with diethanolamine in the presence of carbon monoxide and hydrogen.

11. The process of claim 3, wherein the C₇₋₂₃ hydrocarbon group is substituted with at least one hydroxyl-containing ester group.

12. The process of claim 11, wherein the amide compound is formed by reacting an epoxidized fatty acid or ester thereof with an aminoalcohol to form an amide compound containing at least one epoxide group, and then reacting a hydroxy acid or hydroxy acid precursor with at least one epoxide group of the amide compound to form the hydroxyl-containing ester group.

13. The process of claim 12, wherein the aminoalcohol is monoethanolamine, diethanolamine or a mixture of mono- and diethanolamine.

14. The process of claim 13 wherein the hydroxy acid or hydroxy acid precursor is one or more of lactic acid, glycolic acid, or 2,2-dimethylolpropionic acid.

15. A rigid polyurethane foam made according to the process of claim 1.

16. A hydroxyl-containing amide compound that includes (1) an amide group having at least one hydroxyalkyl group bonded to the nitrogen atom of the amide group, and (2) a branched or straight chain C₇₋₂₃ hydrocarbon group bonded directly to the carbonyl carbon of the amide group, wherein at least one hydroxyl-containing ester group is bonded to the C₇₋₂₃ hydrocarbon group.

17. The amide compound of claim 16 which has an average of at least 3 hydroxyl groups per molecule.

18. A process for preparing a polyol compound having an amide group, comprising (a) forming an amide of (i) a primary or secondary amine compound having at least one hydroxyl-substituted organic group bonded directly or indirectly to the primary or secondary amino nitrogen and (ii) a fatty acid or ester thereof having at least one site of carbon-carbon unsaturation on the fatty acid chain; (b) epoxidizing at least one of said sites of carbon-carbon unsaturation to form an epoxide group and (c) reacting the epoxide group with a hydroxy acid or hydroxy acid precursor to from a pendant, hydroxyl-substituted ester group.

## Patentansprüche

1. Verfahren zur Herstellung eines Polyurethans, mit den folgenden Schritten:
(a) Bilden eines Reaktionsgemisches durch Mischen eines Polyols oder Polyolgemisches mit einer Polyisocyanatverbindung, wobei das Polyol oder Polyolgemisch ein oder mehr Verbindungen enthält, die (1) eine Amidgruppe mit mindestens einer hydroxylhaltigen organischen Gruppe besitzen, die an das Stickstoffatom der Amidgruppe gebunden ist, und (2) eine verzweigt- oder geradkettige C₇₋₂₃-Kohlenwasserstoffgruppe besitzen, die direkt an den Carbonylkohlenstoff der Amidgruppe gebunden ist, wobei die C₇₋₂₃-Kohlenwasserstoffgruppe mit mindestens einer (i) (N-Hydroxyalkyl)aminoalkylgruppe oder (ii) hydroxylhaltigen Estergruppe substituiert ist; und
(b) das Reaktionsgemisch wird solchen Bedingungen unterworfen, dass es zu einem Polyurethan aushärtet.

2. Nach dem Verfahren von Anspruch 1 hergestelltes Polyurethan.

3. Verfahren nach Anspruch 1, wobei das Reaktionsgemisch ferner ein Treibmittel und ein Tensid umfasst, das Polyol oder Polyolgemisch ein durchschnittliches Hydroxyläquivalentgewicht von 100 bis 350 und eine durchschnittliche Hydroxylfunktionalität von mindestens 2,5 besitzt und das Polyurethan ein Polyurethanhartschaum ist.

4. Verfahren nach Anspruch 3, wobei die C₇₋₂₃-Kohlenwasserstoffgruppe mit mindestens einer (N-Hydroxyalkyl)aminoalkylgruppe substituiert ist.

5. Verfahren nach Anspruch 4, wobei mindestens eine Hydroxyalkylgruppe an das Stickstoffatom der Amidgruppe gebunden ist.

6. Verfahren nach Anspruch 5, wobei die an das Stickstoffatom der Amidgruppe gebundene Hydroxyalkylgruppe eine Hydroxyethylgruppe ist.

7. Verfahren nach Anspruch 6, wobei die N-(Hydroxyalkyl)aminoalkylgruppe eine N-(Hydroxyalkyl)aminomethylgruppe ist.

8. Verfahren nach Anspruch 7, wobei die N-Hydroxyalkylaminomethylgruppe eine N-(Hydroxyethyl)aminomethylgruppe oder eine N,N-Bis(hydroxyethyl)aminomethylgruppe ist.

9. Verfahren nach Anspruch 8, wobei zwei Hydroxyethylgruppen an das Stickstoffatom der Amidgruppe gebunden sind.

10. Verfahren nach Anspruch 9, wobei die Amidverbindung gebildet wird, indem zunächst eine ungesättigte Fettsäure oder ein Ester davon mit einem Diethanolamin und Monoethanolamin enthaltenden Alkanolamingemisch umgesetzt wird, um eine Amidverbindung zu bilden, und dann die Amidverbindung in Gegenwart von Kohlenmonoxid und Wasserstoff mit Diethanolamin umgesetzt wird.

11. Verfahren nach Anspruch 3, wobei die C₇₋₂₃-Kohlenwasserstoffgruppe mit mindestens einer hydroxylhaltigen Estergruppe substituiert ist.

12. Verfahren nach Anspruch 11, wobei die Amidverbindung gebildet wird, indem eine epoxidierte Fettsäure oder ein Ester davon mit einem Aminoalkohol zu einer Amidverbindung umgesetzt wird, die mindestens eine Epoxidgruppe enthält, und indem dann eine Hydroxylsäure oder ein Hydroxylsäurevorläufer mit mindestens einer Epoxidgruppe der Amidverbindung zu der hydroxylhaltigen Estergruppe umgesetzt wird.

13. Verfahren nach Anspruch 12, wobei der Aminoalkohol Monoethanolamin, Diethanolamin oder eine Mischung von Mono- und Diethanolamin ist.

14. Verfahren nach Anspruch 13, wobei die Hydroxylsäure oder der Hydroxylsäurevorläufer eines oder mehrere von Milchsäure, Glycolsäure oder 2,2-Dimethylolpropionsäure ist.

15. Nach dem Verfahren von Anspruch 1 hergestellter Polyurethanhartschaum.

16. Hydroxylhaltige Amidverbindung, die Folgendes umfasst: (1) eine Amidgruppe mit mindestens einer an das Stickstoffatom der Amidgruppe gebundenen Hydroxyalkylgruppe, und (2) eine verzweigt- oder geradkettige C₇₋₂₃-Kohlenwasserstoffgruppe, die direkt an den Carbonylkohlenstoff der Amidgruppe gebunden ist, wobei mindestens eine hydroxylhaltige Estergruppe an die C₇₋₂₃-Kohlenwasserstoffgruppe gebunden ist.

17. Amidverbindung gemäß Anspruch 16, die im Durchschnitt mindestens 3 Hydroxylgruppen pro Molekül besitzt.

18. Verfahren zur Herstellung einer Polyolverbindung mit einer Amidgruppe, mit den folgenden Schritten: (a) Bilden eines Amids aus (i) einer primären oder sekundären Aminverbindung mit mindestens einer hydroxylsubstituierten organischen Gruppe, die direkt oder indirekt an den primären oder sekundären Aminostickstoff gebunden ist, und (ii) einer Fettsäure oder einem Ester davon mit mindestens einer Stelle der Kohlenstoff-Kohlenstoff-Ungesättigtheit an der Fettsäurekette; (b) Epoxidieren mindestens einer der Stellen der Kohlenstoff-Kohlenstoff-Ungesättigtheit, um eine Epoxidgruppe zu bilden, und (c) Umsetzen der Epoxidgruppe mit einer Hydroxylsäure oder einem Hydroxylsäurevorläufer, um eine hydroxylsubstituierte Esterseitengruppe zu bilden.

## Revendications

1. Procédé de préparation d'un polyuréthane, comportant les étapes suivantes :
a) préparer un mélange réactionnel en mélangeant un polyol ou un mélange de polyols avec un composé de type polyisocyanate, lequel polyol ou mélange de polyols contient un ou plusieurs composé(s) comportant
1) un groupe amide portant, lié à son atome d'azote, au moins un groupe organique hydroxylé,
2) et un groupe hydrocarboné en C₇₋₂₃, à chaîne linéaire ou ramifiée, directement lié à l'atome de carbone du groupe carbonyle du groupe amide, lequel groupe hydrocarboné en C₇₋₂₃ porte, en tant que substituant, au moins
i) un groupe N-(hydroxy-alkyl)-amino-alkyle
ii) ou un groupe ester hydroxylé ;
b) et mettre ce mélange réactionnel dans des conditions telles qu'il durcisse en formant un polyuréthane.

2. Polyuréthane obtenu selon un procédé conforme à la revendication 1.

3. Procédé conforme à la revendication 1, dans lequel le mélange réactionnel contient en outre un agent d'expansion et un agent tensioactif, le polyol ou le mélange de polyols présente un poids moyen d'équivalent hydroxyle de 100 à 350 et un nombre moyen de groupes fonctionnels hydroxyle d'au moins 2,5, et le polyuréthane est à l'état de mousse rigide.

4. Procédé conforme à la revendication 3, dans lequel le groupe hydrocarboné en C₇₋₂₃ porte, en tant que substituant, au moins un groupe N-(hydroxy-alkyl)-amino-alkyle.

5. Procédé conforme à la revendication 4, dans lequel il y a, lié à l'atome d'azote du groupe amide, au moins un groupe hydroxyalkyle.

6. Procédé conforme à la revendication 5, dans lequel le groupe hydroxyalkyle lié à l'atome d'azote du groupe amide est un groupe hydroxyéthyle.

7. Procédé conforme à la revendication 6, dans lequel le groupe N-(hydroxy-alkyl)-amino-alkyle est un groupe N-(hydroxy-alkyl)-aminométhyle.

8. Procédé conforme à la revendication 7, dans lequel le groupe N-(hydroxy-alkyl)-amino-méthyle est un groupe N-(hydroxy-éthyl)-aminométhyle ou un groupe N,N-bis(hydroxy-éthyl)-amino-méthyle.

9. Procédé conforme à la revendication 8, dans lequel il y a, liés à l'atome d'azote du groupe amide, deux groupes hydroxyéthyle.

10. Procédé conforme à la revendication 9, pour lequel on forme le composé à groupe amide en faisant réagir d'abord un acide gras insaturé, ou un ester d'un tel acide, avec un mélange d'alcanolamines contenant de la diéthanolamine et de la monoéthanolamine, pour former un amide que l'on fait ensuite réagir avec de la diéthanolamine, en présence d'hydrogène et de monoxyde de carbone.

11. Procédé conforme à la revendication 3, dans lequel le groupe hydrocarboné en C₇₋₂₃ porte, en tant que substituant, au moins un groupe ester hydroxylé.

12. Procédé conforme à la revendication 11, pour lequel on forme le composé à groupe amide en faisant réagir un acide gras époxydé ou un ester d'un tel acide avec un amino-alcool, pour former un amide comportant au moins un groupe époxyde, et en faisant ensuite réagir un hydroxy-acide ou un précurseur d'hydroxy-acide avec au moins un groupe époxyde de l'amide, pour former le groupe ester hydroxylé.

13. Procédé conforme à la revendication 12, dans lequel l'amino-alcool est de la monoéthanolamine, de la diéthanolamine, ou un mélange de monoéthanolamine et de diéthanolamine.

14. Procédé conforme à la revendication 13, dans lequel l'hydroxy-acide ou le précurseur d'hydroxy-acide est l'un ou plusieurs des acides lactique, glycolique et 2,2-bis(hydroxyméthyl)propionique.

15. Mousse rigide en polyuréthane, obtenue selon un procédé conforme à la revendication 1.

16. Composé de type amide hydroxylé, qui comporte :
1) un groupe amide portant, lié à son atome d'azote, au moins un groupe hydroxy-alkyle,
2) et un groupe hydrocarboné en C₇₋₂₃, à chaîne linéaire ou ramifiée, direc-tement lié à l'atome de carbone du groupe carbonyle du groupe amide,
et dans lequel il y a au moins un groupe ester hydroxylé lié au groupe hydrocarboné en C₇₋₂₃.

17. Composé de type amide conforme à la revendication 16, dans lequel il y a en moyenne au moins 3 groupes hydroxyle par molécule.

18. Procédé de préparation d'un polyol à groupe amide, comportant les étapes suivantes :
a) former un amide à partir
i) d'une amine primaire ou secondaire comportant au moins un groupe organique à substituant(s) hydroxyle, lié directement ou indirectement à l'atome d'azote du groupe amino primaire ou secondaire,
ii) et d'un acide gras ou d'un ester d'acide gras, dont la chaîne d'acide gras comporte au moins un site d'insaturation carbone-carbone ;
b) époxyder au moins l'un desdits sites d'insaturation carbone-carbone, pour former un groupe époxyde ;
c) et faire réagir ce groupe époxyde avec un hydroxy-acide ou un précurseur d'hydroxy-acide, pour former un groupe ester hydroxylé pendant.
